# FASCICULE DE BREVET EUROPEEN

(11) **EP 3 125 879 B1**
(45) Date de publication et mention de la délivrance du brevet: **22.07.2020**
(21) Numéro de dépôt: 15713739.9
(22) Date de dépôt: 31.03.2015
(51) Int. Cl.: A61K 31/19, A61K 31/215, A61P 43/00

(54) **COMPOSITIONS À BASE DE PHÉROMONES DESTINÉES À TRAITER LES PROBLÈMES COMPORTEMENTAUX OU MÉDICAUX LIÉS AU STRESS DES MAMMIFÈRES NON HUMAINS**
ZUSAMMENSETZUNGEN AUF PHEROMONBASIS ZUR BEHANDLUNG VON STRESSBEDINGTEN VERHALTENSPROBLEMEN ODER MEDIZINISCHEN PROBLEMEN VON NICHT-HUMANEN SÄUGETIEREN
PHEROMONE COMPOSITIONS INTENDED FOR TREATING STRESS-RELATED BEHAVIOURAL OR MEDICAL DISORDERS IN NON-HUMAN MAMMALS

(30) Priorité: 31.03.2014 EP 14162752
(43) Date de publication de la demande: 08.02.2017
(73) Titulaire: Ceva Santé Animale, 33500 Libourne (FR)
(72) Inventeur: LACOSTE, Sandrine, F-33360 Carignan de Bordeaux (FR)
(74) Mandataire: Cabinet Becker et Associés
(86) Numéro de dépôt international: PCT/EP2015/057031
(87) Numéro de publication internationale: WO 2015/150386

(56) Documents cités:
- WO-A1-2009/053347
- WO-A1-2013/043879
- WO-A1-2014/001836
- Anonymous: "FlexiSolv DBE esters", Internet , 30 janvier 2013 (2013-01-30), XP002724069, Extrait de l'Internet: URL:http://www.flexisolv.com/documents/Fle xiSolv DBE esters Technical Data Sheet.pdf [extrait le 2014-05-08]
- Anonymous: "Rhodiasolv", Internet , septembre 2003 (2003-09), XP002724070, Extrait de l'Internet: URL:http://www.rhodia.com/en/binaries/Nove care_Rhodiasolv_brochure_2008_EN.pdf [extrait le 2014-05-08]

## Description

La présente invention concerne généralement le domaine du comportement animal, et particulièrement des compositions de phéromones et des méthodes d'utilisation de ces compositions pour modifier le comportement de mammifères non humains. Plus particulièrement, la présente invention concerne une composition à base de phéromones comprenant au moins un solvant porteur et de préférence un mélange d'acides gras, et/ou de dérivés, de préférence de dérivés esters, en particulier esters de méthyle, de ceux-ci. Cette composition à base de phéromones est susceptible d'être administrée à des mammifères non humains par diffusion dans l'air ambiant, pour le traitement de symptômes liés aux stress ou à l'anxiété desdits mammifères non humains.

Une phéromone est un composé de signalisation chimique ou un mélange de substances naturellement produites par de nombreux animaux, notamment des mammifères non humains, qui suscite une réponse comportementale prévisible et spécifique à un autre membre de la même espèce.

Plusieurs types de phéromones, principalement chez les insectes, ont été identifiés comme des phéromones sexuelles, des phéromones de piste, des phéromones épidéictiques ou d'espacement, des phéromones grégaires, des phéromones d'alarme, des phéromones de régulation sociale, etc.

Un nombre croissant d'études et de produits mis sur le marché tendent à montrer l'influence des phéromones ou de composés mimant l'action des phéromones dans la modification du comportement d'animaux non humains, particulièrement les mammifères, comme par exemple les canidés, les félidés et/ou les équidés.

Ainsi, la demande de brevet EP0724832 décrit que l'association de la valériane à un mélange d'acides gras, comme par exemple l'acide oléique, l'acide caproïque, l'acide palmitique, l'acide azélaïque, l'acide pimélique, l'acide 5-aminovalérique, l'acide n-butyrique, l'acide alpha-méthylbutyrique, l'acide propionique, l'acide 5b-cholestane 3bol p-hydroxyphénylacétique et l'acide butyrique, ou de dérivés esters, en particulier esters de méthyle, de ceux-ci, empêche les chats d'uriner dans un endroit marqué.

La demande de brevet EP1047415 décrit des compositions pour le traitement du stress, de l'anxiété et/ou d'un comportement agressif chez un mammifère, caractérisées par le mélange d'au moins trois acides gras : l'acide palmitique, l'acide linoléique et l'acide oléique, ou des dérivés de ceux-ci.

La demande de brevet EP2515647 décrit une composition phéromonale permettant de modifier le comportement d'un animal comprenant un mélange d'au moins 30% de squalène, au moins 10% d'acide linoléique, et au moins 1% de 1-docosanol, les pourcentages étant en poids, dans lequel la composition phéromonale ne contient pas d'acide palmitique ou d'acide oléique.

La demande de brevet WO2013043879 décrit une composition permettant de modifier le comportement d'un animal, comprenant une interomone qui est plus particulièrement décrite comme étant le 2-méthylbut-2-énal.

Enfin, la demande de brevet EP2271204 décrit une composition de phéromones apaisante pour le chien, nommée phéromone « H », comprenant de l'acide linoléique, de l'acide caprique, de l'acide pentadécylique, de l'acide azélaïque, de l'acide pimélique, de l'acide laurique, de l'acide myristique, du cholestérol, du Cl-docosanol, du squalène et du BHT (butylhydroxytoluène).

Ces compositions à base de phéromones conventionnellement utilisées pour prévenir ou traiter les problèmes comportementaux ou médicaux liés au stress des mammifères non humains sont préférentiellement des mélanges d'acides gras, ou de dérivés de ceux-ci, qui sont diffusés dans l'air ambiant à partir de formes solides, d'émulsions ou de formes liquides.

Ces modes d'administration ont pour but de placer ou distribuer la composition phéromonale dans l'environnement de l'animal, soit par incorporation de la composition dans un appareil pour diffusion, soit par l'application, comme par exemple la pulvérisation via un aérosol ou un spray, de la composition sur des surfaces dans l'environnement de vie de l'animal.

Le mode d'administration préféré est la forme liquide comprenant au moins un solvant porteur afin de permettre une application via par exemple une pompe ou par diffusion, par l'utilisation en particulier de diffuseurs électriques.

Les solvants porteurs convenables sont généralement connus dans le métier et sont reconnus pour inclure des diluants organiques lipophiles, les alcools, l'éthylène glycol, le propylène glycol, le dipropylène glycol, l'éther, le chloroforme, le benzène, le disulfure de carbone, les huiles, de l'eau ou des solvants hydrocarbonés, en particulier des dérivés du pétrole. Par exemple, la demande de brevet WO2013043896 décrit une composition phéromonale dissoute dans un alcool approprié comme l'éthanol, le propanol, l'isopropanol, le butanol, le pentanol, l'hexanol, l'heptanol, l'octanol ou l'alcool phényléthylique pour réaliser une forme liquide capable d'être pulvérisée par une pompe ou un spray.

Des microémulsions pulvérisables sont décrites dans le brevet FR2795960 et sont caractérisées en ce qu'elles comprennent des acides gras, des tensioactifs de préférence anioniques ou non-ioniques, et jusqu'à 30% d'agents co-tensioactifs tels que des alcools en C1-C12 linéaires ou ramifiés.

De façon inattendue, la Demanderesse a mis en évidence de nouveaux solvants porteurs qui présentent des avantages notables pour une administration par diffusion dans l'air ambiant des compositions à base de phéromones.

### Résumé de l'invention

La présente invention concerne une composition à base de phéromones comprenant au moins un solvant porteur caractérisé par les propriétés suivantes :
- une pression de vapeur (VP) comprise entre 1 et 10 Pa (mesurée à 20 ou 25°C), et
- un point éclair supérieur ou égal à 95°C, dans laquelle le solvant porteur comprend au moins un ester dibasique de formule (I) (voir plus bas).

Un autre objet de l'invention est une composition selon l'invention, pour une utilisation dans la prévention et/ou le traitement d'au moins un problème comportemental et/ou médical lié au stress chez un mammifère non humain.

Un autre objet de l'invention est un dispositif de diffusion, adapté à la diffusion dans l'air ambiant d'une composition selon l'invention.

Un autre objet de l'invention est un kit comprenant une composition et/ou un dispositif selon l'invention et optionnellement une notice d'utilisation, destiné à prévenir et/ou traiter les problèmes comportementaux ou médicaux liés au stress des mammifères non humains.

### Description des figures

- Figure 1 :: Variation de la diffusion de différents solvants en fonction du temps.
- Figure 2 :: Diffusion de compositions selon l'invention à une puissance de 2,8 W.
- Figure 3 :: Diffusion d'une composition selon l'invention à différentes puissances, et comparaison avec une composition témoin diffusée à 5,6W.
- Figure 4 :: Stabilité du profil de la diffusion d'une composition selon l'invention sur deux (2) mois.

### Description détaillée de l'invention

Un premier objet de l'invention concerne une composition à base de phéromones comprenant au moins un solvant porteur caractérisé par les propriétés suivantes :
- une pression de vapeur (VP) comprise entre 1 et 10 Pa (mesurée à 20 ou 25°C),
- un point éclair supérieur ou égal à 95°C (coupelle fermée), dans laquelle le solvant porteur comprend au moins un ester dibasique de formule (I) (voir plus bas).
Le solvant porteur selon l'invention n'est pas un solvant dérivé du pétrole.

Selon un mode préféré, le solvant porteur est un produit synthétique biodégradable. Le solvant porteur de la composition à base de phéromone selon l'invention comprend au moins un ester dibasique de formule (I) : 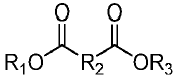 Dans la formule (I) R₁, R₂ et R₃ sont indépendamment des groupements alkyles, linéaires ou ramifiés, comprenant de 1 à 10 atomes de carbone.

Cette composition à base de phéromones est susceptible de prévenir et/ou traiter au moins un problème comportemental et/ou médical lié au stress chez un mammifère non humain.

### Phéromones

Par « composition à base de phéromones », on entend dans la présente invention toute composition comprenant au moins une substance sécrétée par l'organisme d'une espèce particulière qui provoque une réaction prévisible sur un autre individu de la même espèce ou d'une autre espèce. Ladite substance peut être d'origine naturelle ou synthétique. Ainsi, plus généralement, ladite substance est au moins un composé qui mime une substance sécrétée par l'organisme d'un mammifère non humain et est susceptible d'induire un changement de comportement chez un mammifère non humain. Ladite substance peut servir, par exemple, comme attractif spécifique, répulsif spécifique, communicateur social et/ou stimulant sexuel. De préférence, les phéromones sont des acides gras ou dérivés de ceux-ci. Ainsi, la composition à base de phéromones comprend de préférence au moins un acide gras ou un de ses dérivés, de préférence un dérivé ester, en particulier ester de méthyle.

Par « acides gras », on entend selon l'invention, des acides monocarboxyliques et dicarboxyliques à chaîne hydrocarbonée, saturés ou insaturés, linéaires ou ramifiés, actifs, susceptibles de prévenir et/ou traiter les problèmes comportementaux ou médicaux liés au stress des mammifères non humains. Ces acides gras sont de préférence en C4-C22. Ceux-ci sont notamment choisis parmi l'acide propanoïque (propionique), l'acide butanoïque (butyrique), l'acide pentanoïque (valérique), l'acide hexanoïque (caproïque), l'acide heptanoïque (énanthique), l'acide octanoïque (caprylique), l'acide nonanoïque (pélargonique), l'acide décanoïque (caprique), l'acide undécanoïque (undécylique); l'acide dodécanoïque (laurique), l'acide tridécanoïque (tridécylique), l'acide heptadécanoïque (margarique), l'acide octadécanoïque (stéarique), l'acide éicosanoïque, l'acide arachidonique, l'acide henéicosanoïque (henéicosylique), l'acide tricosanoïque (tricosylique), l'acide tétracosanoïque (lignocérique), l'acide pentacosanoïque (pentacosylique), l'acide hexacosanoïque (cérotique), l'acide heptacosanoïque (heptacosylique), l'acide octacosanoïque (montanique), l'acide nonacosanoïque (nonacosylique), l'acide triacontanoïque (mélissique), l'acide henatriacontanoïque (henatriacontylique), l'acide dotriacontanoïque (lacceroïque), l'acide tritriacontanoïque (psyllique), l'acide tetratriacontanoïque (geddique), l'acide pentatriacontanoïque (céroplastique), l'acide hexatriacontanoïque (hexatriacontylique), l'acide nonanedioïque (azélaïque), l'acide tétradécanoïque (myristique), l'acide pentadécylique (n-pentadécanoïque) et l'acide heptanedioïque (pimélique). Des dérivés de ces acides gras tels que les esters, en particulier les esters de méthyles, et/ou les sels peuvent également être utilisés dans la composition.

Par dérivés des acides gras, on entend tous les dérivés actifs, de préférence volatils, en particulier plus volatils que les acides gras non dérivés. De préférence, les dérivés sont des formes estérifiées, et plus particulièrement des esters de méthyle.

Les compositions selon l'invention peuvent comprendre tout mélange d'acides gras et/ou de leurs dérivés susceptibles de prévenir et/ou traiter au moins un problème comportemental ou médical lié au stress des mammifères non humains.

Dans un mode de réalisation de l'invention, les phéromones sont des acides gras choisis parmi: l'acide oléique, l'acide palmitique, l'acide n-butyrique, l'acide linoléique, l'acide palmitoléique, l'acide caprique, l'acide laurique, l'acide myristique, l'acide pentadécanoïque, l'acide azélaïque, l'acide pimélique, l'acide stéarique, l'acide linolénique, l'acide pentanoïque, l'acide arachidonique, l'acide isobutyrique, l'acide pivalique, l'acide eicosapentonoïque, l'acide tridécanoïque, l'acide docosahexaénoïque, un de leurs dérivés, et un mélange de ceux-ci.

De préférence, au moins un des acides gras présents dans la composition à base de phéromones selon l'invention est l'acide linoléique, l'acide oléique ou l'acide palmitique.

A titre d'exemples de phéromones, sans être exhaustif, on peut citer les mélanges d'acides gras suivants :
- un mélange d'acide oléique et d'acide palmitique ;
- un mélange d'acide oléique et d'acide n-butyrique ;
- un mélange d'acide oléique, d'acide palmitique et d'acide linoléique ;
- un mélange d'acide oléique, d'acide palmitique, d'acide linoléique et d'acide palmitoléique ;
- un mélange d'acide oléique, d'acide palmitique, d'acide azélaique et d'acide pimélique ;
- un mélange d'acide caprique, d'acide laurique, d'acide myristique, d'acide palmitoléique, d'acide palmitique, d'acide linoléique et d'acide oléique ;
- un mélange d'acide oléique, d'acide palmitique, d'acide linoléique et d'acide myristique ;
- un mélange d'acide oléique, d'acide palmitique, d'acide linoléique, d'acide laurique et d'acide myristique ;
- un mélange d'acide oléique, d'acide palmitique, d'acide linoléique, d'acide myristique et d'acide pentadécanoïque ;
- un mélange d'acide oléique, d'acide palmitique, d'acide linoléique, d'acide myristique, d'acide pentadécanoïque et d'acide stéarique ;
- un mélange d'acide oléique, d'acide palmitique, d'acide linoléique, d'acide myristique, d'acide laurique et d'acide pentadécanoïque ;
- un mélange d'acide laurique, d'acide myristique, d'acide pentadécanoïque, d'acide palmitique, d'acide stéarique, d'acide oléique, et d'acide linoléique ; ou
- un mélange d'acide oléique, d'acide azélaïque, d'acide pimélique et d'acide palmitique,
ou un de ces mélanges dans lequel au moins un des acides est remplacé par un de ses dérivés, de préférence dérivés esters, en particulier esters de méthyle.

Les mélanges peuvent comprendre les acides gras précités dans des proportions appropriées connues de l'homme du métier. A titre d'exemples, les mélanges peuvent comprendre, par rapport au poids total du mélange :
- environ 55 à 65% d'acide oléique, et 45 à 35% d'acide palmitique, ou d'un de leurs dérivés respectifs ;
- environ 45% d'acide oléique, 16% d'acide azélaïque, 18% d'acide pimélique, et 21% d'acide palmitique, ou d'un de leurs dérivés respectifs ;
- environ 30% d'acide palmitique, 30% d'acide oléique, et 40% d'acide linoléique, ou d'un de leurs dérivés respectifs ; ou
- environ 30% d'acide palmitique, 40% d'acide linoléique, 10% d'acide palmitoléique, et 20% d'acide oléique, ou d'un de leurs dérivés respectifs.

Dans les compositions à base de phéromones selon l'invention, les acides gras ou leurs dérivés peuvent être présents en proportion adaptée à la diffusion dans l'air ambiant. De préférence, une composition à base de phéromones selon l'invention comprend (fraction phéromonale) entre environ 0,01 et environ 5% d'acides gras et/ou leurs dérivés, de préférence entre environ 0,05 et environ 5%, de préférence entre environ 2 et environ 4%, de préférence entre environ 3 et environ 4%, en particulier environ 3,5%, par rapport au poids total de la composition.

Sauf précision contraire, les pourcentages sont dans le cadre de la présente invention des pourcentages en masse.

La mention « environ » relative à une valeur désigne l'intervalle compris entre plus et moins 10% de ladite valeur.

### Solvant porteur

Les solvants porteurs dans la composition selon l'invention sont des solvants caractérisés par les propriétés suivantes :
- une pression de vapeur (VP) comprise entre 1 et 10 Pa (mesurée à 20 ou 25°C), et
- un point éclair supérieur ou égal à 95°C (coupelle fermée), dans laquelle le solvant porteur comprend au moins un ester dibasique de formule (I) (voir ci-haut).

Le solvant porteur selon l'invention présente une pression de vapeur (VP) comprise entre 1 et 10 Pa (mesurée à 20 ou 25°C), de préférence comprise entre 4 et 8 Pa, elle est avantageusement comprise entre 5 et 7 Pa (comme, par exemple, 6,3 Pa).

La pression de vapeur correspond à celle définie dans la directive 1999/13/CE à l'article 2 de la définition 17) des composés organiques volatiles. Elle est donc mesurée selon les méthodes classiques liées à cette définition. Plus spécifiquement, les solvants porteurs selon la présente invention ne sont pas des composés organiques volatiles (selon la définition 17) de l'article 2 de la directive 1999/13/CE).

Le solvant porteur selon l'invention présente un point éclair supérieur ou égal à 95°C, de préférence comprise entre 96 et 200°C, avantageusement il est de 98 ou de 144°C.

Le point d'éclair en coupelle fermée se mesure généralement à l'aide d'un appareil dit de Pensky-Martens. Un appareil semi-automatique de ce type est constitué d'une coupelle que l'on peut remplir du liquide (en général un hydrocarbure) dont on désire connaître le point d'éclair. On referme ensuite la coupelle. Le couvercle est muni d'un thermomètre dont l'embout se situe au-dessus du liquide dans les vapeurs. L'appareil dispose d'un chauffage qui permet d'élever la température degré par degré. Chaque fois que la température atteint un degré supérieur, une flamme est plongée dans les vapeurs. S'il y a inflammation, c'est que le point d'éclair est atteint, dans le cas contraire l'appareillage continue d'augmenter la température du liquide.

Selon un mode préféré, le solvant porteur selon l'invention n'est pas un solvant dérivé du pétrole. En particulier, le solvant porteur selon l'invention est au moins un composé solvant comprenant, ou consistant (éventuellement essentiellement) en, des atomes de carbone, d'hydrogène et d'oxygène.

Selon un mode préféré, le solvant porteur est un produit synthétique biodégradable.

Par produit synthétique, on entend selon l'invention un produit qui a été obtenu par un procédé partiellement ou de préférence totalement synthétique, et qui n'existe pas dans la nature.

Par « solvant dérivé du pétrole », on entend un solvant comprenant uniquement des atomes de carbone et d'hydrogène.

Par « produit synthétique biodégradable », on entend selon l'invention un produit qui répond à la ligne directrice de l'OCDE n°301 et/ou 310.

En particulier, un produit biodégradable a un taux de biodégradabilité minimum de 60% obtenu selon les tests décrits dans les normes : AFNOR T90-312 et/ou ISO7827, de préférence compris entre 70 et 99% avantageusement il est de 90%.

Le solvant porteur est avantageusement pur, c'est-à-dire qu'il présente un degré de pureté supérieur à 95%.

Par « pureté supérieure à 95% », on entend que le solvant ne comprend pas plus de 5% en masse d'impuretés, c'est-à-dire de composants autres que ceux constitutifs du solvant, avant sa mise en contact avec les autres composants de la composition phéromonale selon l'invention. De préférence, les termes « solvant pur » désignent un solvant comprenant moins de 5 % d'autres composés constitutifs du solvant, d'impuretés et contaminants, plus particulièrement moins de 4 %, moins de 3 %, moins de 2%, et encore plus préférentiellement moins de 1 % d'autres espèces moléculaires, d'impuretés et de contaminants.

Le degré de pureté peut être évalué par toute méthode analytique connue de l'homme du métier, telle que par exemple par HPLC (Chromatographie Haute Performance Liquide), chromatographie en phase gazeuse (CPG), spectrométrie de masse, ou résonnance magnétique nucléaire. Ces méthodes analytiques peuvent, bien entendu, être couplées ou combinées entre elles.

Le solvant utilisé dans la présente invention est avantageusement non classifié dangereux par inhalation.

Par solvant « non classifié dangereux par inhalation », on entend un solvant qui ne porte aucune des phrases de risque suivantes : R20 : Nocif par inhalation, R23 : Toxique par inhalation, et R26 : Très toxique par inhalation. Les phrases de risque sont définies dans l'annexe III de la Directive européenne 67/548/EEC, complétée par la Directive 2001/59/CE.

De préférence, le solvant porteur ne contient aucun monoester carboné de C8 à C18. Avantageusement, le solvant porteur est un solvant sans odeur.

Par « sans odeur », on désigne un composé dont aucune odeur ne peut être perçue par l'utilisateur humain lorsque la composition à base de phéromones est diffusée, de préférence dans l'air ambiant.

Avantageusement, le solvant porteur présente au moins une des propriétés identifiées ci-dessus et avantageusement toutes ces propriétés.

Comme exemples de solvants porteurs selon l'invention, on peut citer notamment les esters dibasiques de formule (I), tels que le diméthyléther d'isosorbide, l'isononanoate d'isononyle, et le carbonate de propylène. On préfère le propylène carbonate ou avantageusement les esters dibasiques.

### Esters dibasiques

Les esters dibasiques selon l'invention sont des composés de formule (I) 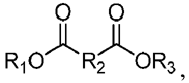 dans laquelle R₁, R₂ et R₃ sont indépendamment des groupements alkyles, linéaires ou ramifiés, comprenant de 1 à 10 atomes de carbone.

Un groupement « alkyle » selon l'invention est un groupement hydrocarboné saturé, linéaire ou ramifié, comprenant de 1 à 10 atomes de carbone.

De préférence, le groupement alkyle comprend de 1 à 4 atomes de carbone. Le groupement alkyle peut notamment être choisi parmi les groupements méthyle, éthyle, n-propyle, isopropyle, n-butyle, isobutyle et tert-butyle. En particulier, le groupement alkyle est un groupement méthyle, éthyle, n-propyle, n-butyle ou isobutyle.

Dans un mode de réalisation, les groupements R₁ et R₃ sont identiques. De préférence, R₁ et R₃ sont deux groupements isobutyle.

Dans un mode de réalisation, R₂ est choisi parmi les groupements éthyle, n-propyle, n-butyle et isobutyle.

Comme exemples d'esters dibasiques, on peut citer notamment les esters de diméthyle tels que le diméthyl 2-méthylpentanedioate et les esters de diisobutyle tels que le diisobutyl hexane dioate, le diisobutyl glutarate et le diisobutyl succinate, et leurs mélanges.

Ces esters dibasiques sont en particulier utilisés à titre de solvants dans la présente invention.

Comme exemples de solvants comprenant au moins un ester dibasique, on peut citer notamment les solvants de la gamme Rhodiasolv®, commercialisés par la société Solvay, par exemple le Rhodiasolv® IRIS (diméthyl 2-méthylpentanedioate) ou le Rhodiasolv® DIB (diisobutyl hexane dioate, diisobutyl glutarate et diisobutyl succinate), ainsi que leurs mélanges en toutes proportions.

De façon préférée, le solvant comprenant au moins un ester dibasique est un mélange de diisobutyl hexane dioate, diisobutyl glutarate et diisobutyl succinate (comme par exemple dans le produit Rhodiasolv® DIB).

Dans les compositions selon l'invention, le solvant porteur, et en particulier le ou les esters dibasiques, peut être présent en toute proportion adaptée à la diffusion dans l'air ambiant. De préférence, une composition selon l'invention comprend entre environ 50 et environ 99,9% de solvant porteur, et en particulier d'ester(s) dibasique(s), de préférence entre environ 75 et environ 99,5%, de préférence entre environ 90 et environ 99%, de préférence entre environ 95 et environ 97%, en particulier environ 96,5%, exprimé en pourcentage de masse par rapport au poids total de la composition.

### Autres composants

Les compositions selon l'invention peuvent contenir éventuellement d'autres composés supplémentaires de ceux qui ont été décrits ci-dessus, en particulier en plus des acides gras et des esters dibasiques tels que définis ci-dessus.

Ces composants supplémentaires, bien connus de l'homme de l'art, peuvent être notamment des solvants, des agents propulseurs, des parfums, des composés attractants, des alcools gras, des stéroides, des antioxydants et/ou des terpènes.

Les solvants peuvent être des diluants organiques lipophiles, les alcools, l'éthylène glycol, le propylène glycol, le dipropylèneglycol, l'éther, le chloroforme, le benzène, le disulfure de carbone, les huiles, y compris les liquides et les huiles non volatiles et instables ou l'eau. On peut également citer comme autres solvants les triglycérides en C8 à C10, le décanol, le citrate de triéthyle, le glycofurol (tétraéthylène glycol), l'acide néopentanoïque, l'acide isononanoïque, et leurs esters, comme le néopentanoate d'isodécyle, l'isononanoate d'octyle, les esters d'isopropanol, d'acide myristique, d'acide citrique, d'acide sébacique, comme par exemple le myristate d'isopropyle, le sébaçate de diisopropyle.

Les agents propsulseurs pourront être des chlorofluorocarbures (CFC) tels que le trichloromonofluorométhane, le dichlorodifluorométhane, le dichlorotétrafluoroéthane et les hydrochlorofluorocarbones (HCFC) ou des hydrofluorocarbures (HFC) tels que le chlorodifluorométhane, le trifluoromonofluoroéthane, le chlorodifluoroéthane, le difluoroéthane, et l'heptafluoropropane, les hydrocarbures tels que le propane, le butane, et l'isobutène, et les gaz comprimés tels que l'azote, le dioxyde de carbone et l'oxyde nitreux.

Les parfums pourrons être des essences florales, des fleurs d'agrumes, de l'huile, des extraits de conifères ou des épices.

Les composés attractants sont des huiles essentielles comme par exemple la valériane ou l'huile de cataire.

Un alcool gras est par exemple le 1-docosanol, un stéroïde, le cholestérol, un antioxydant peut être un antioxydant lipophile comme le BHT (butylhydroxytoluène) et un terpène pourrait être le squalène.

L'homme du métier est à même d'ajuster la nature et la quantité de chaque composé supplémentaire dans la composition selon l'invention en fonction des caractéristiques désirées pour la composition phéromonale.

### Les problèmes comportementaux ou médicaux liés au stress des mammifères non humains

Il est reconnu que le stress peut générer chez les mammifères non humains de nombreux problèmes médicaux ou de comportement. Les sources communément reconnues du stress et /ou de la peur chez les mammifères non humains comprennent par exemple l'arrivée dans un nouveau lieu, le sevrage, la séparation avec la mère en vue d'adoption et/ou de changement de lieu, les transports (notamment dans les véhicules motorisés), l'ennui, le manque d'exercice, l'anxiété liée à la séparation, des bruits forts, la surpopulation, les événements qui induisent l'animal à par exemple aboyer, sauter ou mendier, mais aussi l'anxiété liée à la présence de nouvelles personnes ou de nouveaux mammifères, ou encore l'anxiété liée aux visites chez le vétérinaire et tout autre événement non routinier dans la vie du mammifère non humain.

Les mammifères non-humains qui sont stressés suite à l'exposition à de tels événements ou conditions vont généralement présenter des symptômes comportementaux et/ou développer des problèmes médicaux liés au stress hautement indésirables. Les comportements indésirables sont généralement reconnus et les plus fréquemment rencontrés sont le marquage urinaire, l'agressivité, les griffades, les excès de toilettage, les conflits territoriaux, la diminution des jeux et des contacts sociaux, l'hyperagitation ou la prostration, les destructions. Les problèmes médicaux liés au stress et/ou à la peur sont généralement reconnus et les plus fréquemment rencontrés sont la cystite, les problèmes dermatologiques et notamment le léchage excessif, l'alopécie, l'anorexie, l'obésité, la diarrhée et les infections respiratoires.

### Les mamifères non humains

Par « mammifères non humains », on entend les mammifèrs domestiques, de ferme ou de zoo, et plus particulièrement les félidés, les canidés et/ou les équidés.

### Modes d'administration

L'efficacité de la composition selon l'invention peut être obtenue par exemple par administration dans l'environnement des mammifères non-humains grâce par exemple à une pulvérisation d'aérosol ou de spray comprenant la composition, voire par la diffusion dans l'air ambiant de la composition via un diffuseur liquide. Dans tous les cas, la composition est suffisamment volatile pour que l'animal puisse l'inhaler et donc être exposé à une quantité suffisante de la composition pour produire un effet comportemental notable. Par exemple, une réduction des comportements extérieurs indésirables peut être facilement vérifiable, comme par exemple la réduction notable de son agressivité, griffade, aboiements et / ou saut et peut être complétée par l'observation d'autres indicateurs physiques de stress comme la fréquence cardiaque, les changements de poids, et la sécrétion d'hormones de stress comme comme le cortisol.

La composition selon l'invention peut aussi être utilisée pour prévenir l'apparition de comportements indésirables. Dans ce cas, la composition de la présente invention peut être utilisée pour induire un état temporaire de baisse d'activité, une diminution de l'excitabilité afin de permettre au mammifère non humain de mieux supporter le stress à venir comme par exemple un feu d'artifice, un déplacement ou l'arrivée d'un autre animal.

Le mammifère non-humain sera exposé à la composition de l'invention par tout procédé permettant son inhalation pendant une période de temps suffisante nécessaire à l'observation d'une modification du comportement de la cible, tel que déterminé d'après les observations du comportement. Typiquement, en fonction de la voie d'administration choisie et de la situation génératrice du stress, le mammifère non-humain sera exposé à la composition de l'invention sur une période d'au moins une seconde à quelques mois mais dans tous les cas pour toute période de temps nécessaire pour obtenir un effet comportemental satisfaisant. Par exemple, un animal souffrant d'une anxiété induite temporairement comme par exemple les feux d'artifice, peut exiger une exposition à la composition de l'invention avant, pendant et/ou après l'événement anxiogène pour soulager l'anxiété et le(s) comportement(s) associé(s). En revanche, un animal exposé à un stimulus stressant pour une période plus longue et continuelle, comme par exemple un animal exposé à un nouvel animal de compagnie à la maison, peut bénéficier d'une exposition régulière à la composition de l'invention pendant une période prolongée.

Le mode d'adminsitration préférentiel de la composition de l'invention est une diffusion dans l'air ambiant, laquelle est plus particulièrement réalisée au moyen d'un diffuseur électrique.

Un objet de l'invention est un dispositif de diffusion dans l'air ambiant, adapté à la diffusion dans l'air ambiant d'une composition à base de phéromones selon l'invention. Dans un mode de réalisation, le dispositif de diffusion est un diffuseur, en particulier un diffuseur électrique.

De préférence, le diffuseur est un diffuseur à recharge liquide, c'est-à-dire qu'il s'utilise avec une recharge liquide. La recharge liquide contient une composition selon l'invention telle que définie ci-avant.

De préférence, le diffuseur est adapté pour une diffusion de la composition selon l'invention pendant une durée d'au moins un mois (environ 30 jours).

Le diffuseur, de préférence le diffuseur électrique, selon l'invention permet d'obtenir de préférence une température de l'élément de chauffage des compositions à base de phéromones qui est strictement inférieure à 130°C, notamment inférieure ou égale à 120°C, en particulier inférieure ou égale à 100°C.
Les éléments de chauffage sont bien connus de l'homme du métier et sont par exemple appliqués pour diffuser des substances volatiles comme cela est décrit dans le brevet EP1714662.

Un autre objet de l'invention est un kit comprenant une composition à base de phéromones selon l'invention et/ou un dispositif selon l'invention, et optionnellement une notice d'utilisation, destiné à prévenir et/ou traiter les problèmes comportementaux ou médicaux liés au stress des mammifères non humains.

Un dernier objet de l'invention est un procédé de diffusion d'une composition selon l'invention, comprenant la diffusion dans l'air ambiant de ladite composition. De préférence, le procédé de diffusion comprend l'utilisation d'un dispositif ou d'un kit selon l'invention. De préférence, la diffusion se fait par chauffage, en particulier selon les températures spécifiées ci-dessus.

### Avantages des compositions, dispositifs, kits et procédés selon l'invention

Les compositions phéromonales selon l'invention présentent une variabilité de diffusion nettement réduite par rapport à des compositions comprenant des solvants porteurs hydrocarbonés, notamment par rapport à des compositions d'acides gras ayant comme seuls solvants porteurs des solvants dérivés hydrocarbonés de pétrole en C14-C19.

En outre, l'utilisation de solvants tels que définis dans la présente invention, notamment d'esters dibasiques, permet de réduire de façon conséquente la puissance électrique du diffuseur et la température de l'élémént de chauffage nécessaires à la diffusion des compositions d'acides gras. Ainsi, la diffusion des compositions selon la présente invention pourra se faire avec des diffuseurs caractérisés par des puissances inférieures ou égales à 5,6 W, et plus particulièrement par des puissances inférieures à 3 W.
De même, la diffusion des compositions selon la présente invention pourra se faire par des températures de l'élement de chauffage inférieures à 130°C, notamment inférieures ou égales à 120°C, en particulier inférieures ou égales à 100°C. La diminution de la puissance et de la température des éléments de chauffage permet de réduire en conséquence la température d'échauffement du dispositif, réduisant par là-même l'odeur de brûlé éventuelle et diminuant ainsi les risques perçus de brûlures pour l'utilisateur.

Les exemples ci-dessous sont fournis à titre uniquement illustratif, de l'invention.

### Exemples

### Exemple 1 : Diffusion de compositions phéromonales selon l'invention

Dans un diffuseur électrique à 5,6W sont placés 48ml de solvant à tester. La quantité de solvant diffusé est mesurée pendant 30 jours après le branchement du diffuseur, et la moyenne de diffusion du produit par jour est calculée et résumée dans le tableau 1 ci-dessous:

**Tableau 1**

| Solvant | Diffusion produit (g/jour) |
|---|---|
| Témoin (Solvant issu de distillat de pétrole) | 2 |
| Isopropyl myristate | 1 |
| Diisopropyl sébacate | 1 |
| Isononyl isononanoate | 2 |
| Glycofurol | 0.5 |
| Triethyl citrate | 1 |
| Ester dibasique | 4 |
| Propylène carbonate | 4 |
| Isosorbide dimethyl ether | 2 |

### Exemple 2 : Variation de la diffusion pour une composition à base de phéromones comprenant un solvant ester dibasique

La capacité de diffusion de divers solvants a été évaluée en fonction de la puissance du diffuseur et de la température des éléments de chauffage du diffuseur.

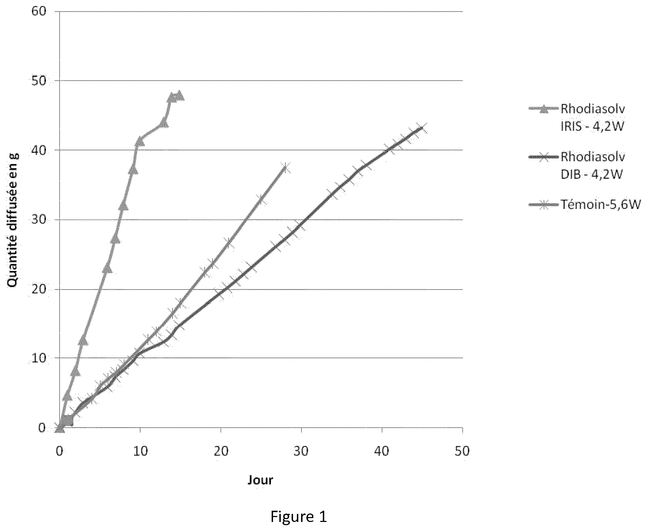

Les conditions sont résumées dans le tableau 2 ci-dessous. Les résultats sont présentés sur le graphe de la figure 1.

**Tableau 2**

| Solvant | Témoin (Solvant issu de distillat de pétrole) | Rhodiasolv® IRIS | Rhodiasolv® DIB |
|---|---|---|---|
| Puissance | 5,6W | 4,2W | 4,2W |
| Température | 130°C | 100°C | 100°C |

Les solvants ont la composition suivante :
Solvant issu de distillat de pétrole est un solvant hydrocarboné issu d'un distillat de pétrole en milieu hydrotraité.
Rhodiasolv® IRIS (vendu et commercialisé par Solvay): diméthyl 2-méthylpentanedioate.
Rhodiasolv® DIB (vendu et commercialisé par Solvay): diisobutyl hexane dioate, diisobutyl glutarate et diisobutyl succinate.

Le graphe de la figure 1 illustre que la diffusion de solvants esters dibasiques à une température de l'élément de chauffage et des puissances de diffuseur inférieures est comparable à celle observée dans les conditions usuelles de diffuseur de composés volatils (solvant issu de distillat de pétrole à 130°C avec une puissance de 5,6W).

### Exemple 3 : Etude du coefficient de variation d'une composition selon l'invention

Le coefficient de variation (CV) d'une composition selon l'invention comprenant 3,5% d'actifs, 19,3% de Rhodiasolv® DIB et 77,2% de Rhodiasolv® IRIS en volume sur 3 diffuseurs à 2,8 W a été étudié chaque jour pendant 10 jours (tableau 3). A titre de comparaison, on fournit le coefficient de variation d'une composition, comprenant 3,5% d'actifs et un solvant issu de distillat de pétrole comme seul solvant, obtenu sur 6 diffuseurs à 5,6W (tableau 4). Les actifs correspondent au mélange d'esters d'acides gras décrit dans la composition 4 de l'exemple 5.

**Tableau 3**

| **Jours** | 1 | 2 | 3 | 6 | 10 |
|---|---|---|---|---|---|
| **Quantité moyenne diffusée par jour (g)** | 2,017 | 2,012 | 2,10 | 1,67 | 1,59 |
| **CV (%)** | **4,85** | **2,61** | **0,84** | **6,345** | **3,08** |

**Tableau 4**

| **Jours** | 1 | 2 | 3 | 6 | 10 |
|---|---|---|---|---|---|
| **Quantité moyenne diffusée par jour (g)** | 1,73 | 1,64 | 1,58 | 1,7 | 1,47 |
| **CV(%)** | **11,1** | **19,2** | **20,7** | **20,8** | **17,7** |

Les coefficients de variation sont clairement inférieurs pour la composition selon l'invention, même en utilisant seulement 3 diffuseurs à 2,8W, que ceux obtenus pour la composition comprenant un solvant issu de distillat de pétrole.

### Exemple 4 : Etude de la diffusion obtenue à différentes puissances de diffuseur

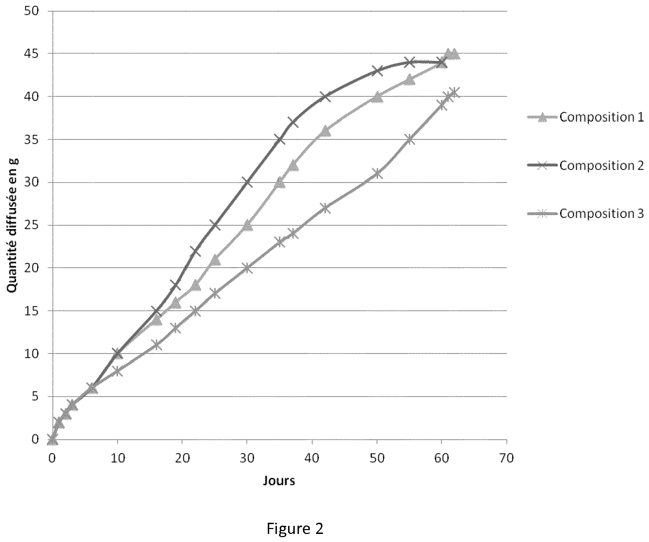

La perte de masse obtenue en fonction du temps de compositions selon l'invention à une puissance de 2,8 W est représentée à la figure 2. Les compositions sont les suivantes (% en volume) :
- Composition 1 : 3,5% d'actifs, 48,25% de Rhodiasolv® DIB, 48,25% de Rhodiasolv® IRIS ;
- Composition 2 : 3,5% d'actifs, 19,3% de Rhodiasolv® DIB, 77,2% de Rhodiasolv® IRIS ;
- Composition 3 : 3,5% d'actifs, 28,95% de Rhodiasolv® DIB, 67,55% de Rhodiasolv® IRIS.
Les actifs correspondent au mélange d'esters d'acides gras décrit dans la composition 4 de l'exemple 5.

Les graphes de la figure 2 montrent qu'une très bonne diffusion peut être obtenue en utilisant seulement une puissance de 2,8 W.

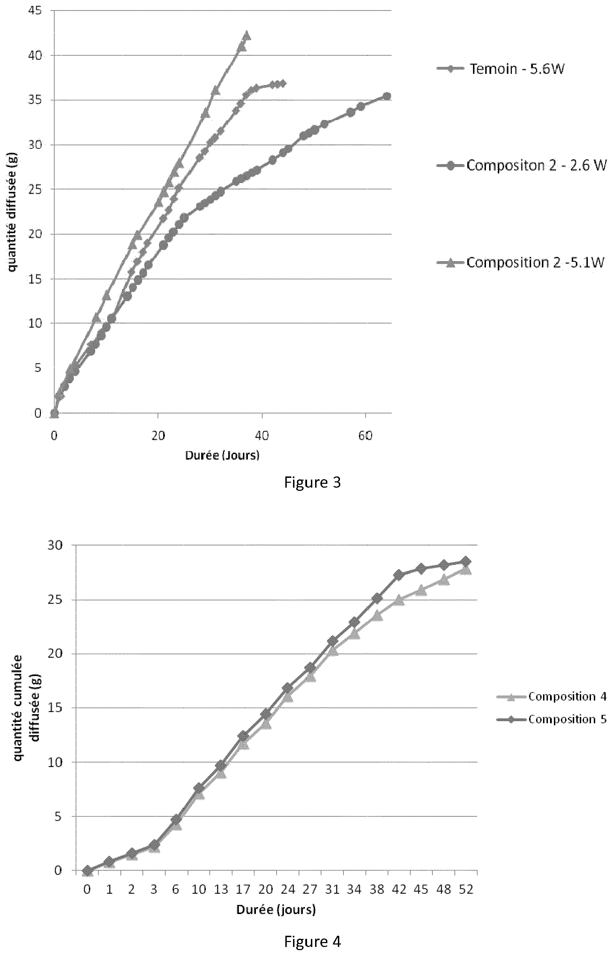

De même, la figure 3 présente les courbes de diffusion obtenues pour la composition 2 selon l'invention à 2,6W et 5,1W, ainsi que la courbe de diffusion obtenue pour une composition comprenant comme solvant un solvant issu de distillat de pétrole, à 5,6W (Témoin 5,6W). Les diffusions obtenues pour les compositions selon l'invention à 2,6W et 5,1W sont comparables avec celles obtenues avec un solvant issu de distillat de pétrole à 5,6W. Par conséquent, il est possible en utilisant les compositions selon l'invention d'obtenir une diffusion équivalente à celle observée avec un diffuseur de 5,6W avec une puissance plus faible, donc à coût énergétique moindre.

### Exemple 5: Compositions selon l'invention

| Composition 4 | en grammes |
|---|---|
| Mélange d'esters d'acides gras comprenant : | |
| acide linoléique | |
| acide oléique | |
| acide stéarique | 3,5 |
| acide palmitique | |
| acide pentadécanoïque | |
| acide myristique | |
| acide laurique | |
| Rhodiasolv® DIB | QSP 100 mL |

### Exemple 6: Stabilité du profil de diffusion d'une composition selon l'invention sur deux (2) mois

La diffusion par un diffuseur d'une puissance de 3W de la composition selon l'exemple 5 (Composition 4) a été comparée à celle d'une composition comprenant les mêmes ingrédients et ayant été stockée pendant 14 mois à 25°C et 60% d'humidité résiduelle (Composition 5).

Les résultats sont présentés à la figure 4.
Les graphes démontrent clairement que les propriétés de diffusion et d'évaporation de la composition selon l'invention sont conservées au cours du temps. Cette composition est donc stable au stockage.

## Revendications

1. Composition à base de phéromones comprenant au moins un solvant porteur **caractérisé par** les propriétés suivantes :
- une pression de vapeur (VP) comprise entre 1 et 10 Pa (mesurée à 20 ou 25°C), et
- un point éclair supérieur ou égal à 95°C,, dans laquelle le solvant porteur comprend au moins un ester dibasique de formule (I) 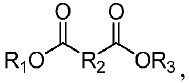 dans laquelle R₁, R₂ et R₃ sont indépendamment des groupements alkyles, linéaires ou ramifiés, comprenant de 1 à 10 atomes de carbone.

2. Composition selon la revendication 1, dans laquelle R₁ et R₃ sont identiques.

3. Composition selon la revendication 2, dans laquelle R₁ et R₃ sont des groupements isobutyle.

4. Composition selon l'une quelconque des revendications 1-3, dans laquelle l'ester dibasique est choisi parmi le diméthyl 2-méthylpentanedioate, le diisobutyl hexane dioate, le diisobutyl glutarate, le diisobutyl succinate et au moins un de leurs mélanges.

5. Composition selon l'une quelconque des revendications 1 à 4, dans laquelle la composition comprend entre 50 et 99,9% de solvant porteur, en particulier d'ester dibasique. en poids par rapport au poids total de la composition.

6. Composition selon l'une quelconque des revendications 1 à 4, dans laquelle la composition comprend entre 95 et 97% de solvant porteur, en particulier d'ester dibasique, en poids par rapport au poids total de la composition.

7. Composition selon l'une quelconque des revendications 1 à 4, dans laquelle la composition comprend 96,5% de solvant porteur, en particulier d'ester dibasique, en poids par rapport au poids total de la composition

8. Composition selon la revendication 1, dans laquelle les phéromones de ladite composition comprennent au moins un acide gras ou un dérivé, de préférence un dérivé ester, en particulier ester de méthyle, de celui-ci.

9. Composition selon la revendication 8, dans laquelle l'acide gras est choisi parmi : l'acide oléique, l'acide palmitique, l'acide n-butyrique, l'acide linoléique, l'acide palmitoléique, l'acide caprique, l'acide laurique, l'acide myristique, l'acide pentadécanoïque, l'acide stéarique, l'acide azélaïque, l'acide pimélique, l'acide linolénique, l'acide pentanoïque, l'acide arachidonique, l'acide isobutyrique, l'acide pivalique, l'acide eicosapentonoïque, l'acide tridécanoïque, l'acide docosahexaénoïque et un mélange de ceux-ci.

10. Composition selon l'une quelconque des revendications 8 et 9, ladite composition comprenant 0,01% à environ 5% en poids d'acide gras ou de dérivé de celui-ci par rapport au poids total de la composition.

11. Composition selon l'une quelconque des revendications 1 à 10, pour une utilisation dans la prévention et/ou le traitement d'au moins un problème comportemental ou médical lié au stress d'un mammifère non humain.

12. Composition pour une utilisation selon la revendication 11, dans laquelle le problème comportemental lié au stress est choisi parmi le marquage urinaire, l'agressivité, les griffades, les excès de toilettage, les conflits territoriaux, la diminution des jeux et des contacts sociaux, l'hyperagitation, la prostration et les destructions.

13. Composition pour une utilisation selon la revendication 11, dans laquelle le problème médical lié au stress est choisi parmi la cystite, les problèmes dermatologiques, l'alopécie, l'anorexie, l'obésité, la diarrhée et les infections respiratoires.

14. Kit comprenant une composition selon l'une quelconque des revendications 1 à 10 et/ou un dispositif adapté à la diffusion dans l'air ambiant de ladite composition, et optionnellement une notice d'utilisation, destiné à prévenir et/ou traiter au moins un problème comportemental ou médical lié au stress chez un mammifère non humain.

## Patentansprüche

1. Zusammensetzung auf Pheromonbasis, umfassend wenigstens ein Trägerlösemittel, **gekennzeichnet durch** die folgenden Eigenschaften:
- einen Dampfdruck (VP) zwischen 1 und 10 Pa (gemessen bei 20 oder 25°C), und
- einen Flammpunkt höher oder gleich 95°C, wobei das Trägerlösemittel wenigstens einen dibasischen Ester der Formel (I) 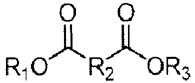 umfasst, wobei R₁, R₂ und R₃ unabhängig voneinander lineare oder verzweigte Alkylgruppen sind, die 1 bis 10 Kohlenstoffatome umfassen.

2. Zusammensetzung gemäß Anspruch 1, wobei R₁ und R₃ identisch sind.

3. Zusammensetzung gemäß Anspruch 2, wobei R₁ und R₃ Isobutylgruppen sind.

4. Zusammensetzung gemäß einem der Ansprüche 1 bis 3, wobei der dibasische Ester aus Dimethyl-2-methylpentandioat, Diisobutylhexandioat, Diisobutylglutarat, Diisobutylsuccinat und wenigstens einem ihrer Gemische ausgewählt ist.

5. Zusammensetzung gemäß einem der Ansprüche 1 bis 4, wobei die Zusammensetzung zwischen 50 und 99,9 Gew.-% Trägerlösemittel, insbesondere dibasischen Ester bezogen auf das Gesamtgewicht der Zusammensetzung umfasst.

6. Zusammensetzung gemäß einem der Ansprüche 1 bis 4, wobei die Zusammensetzung zwischen 95 und 97 Gew.-% Trägerlösemittel, insbesondere dibasischen Ester bezogen auf das Gesamtgewicht der Zusammensetzung umfasst.

7. Zusammensetzung gemäß einem der Ansprüche 1 bis 4, wobei die Zusammensetzung 96,5 Gew.-% Trägerlösemittel, insbesondere dibasischen Ester bezogen auf das Gesamtgewicht der Zusammensetzung umfasst.

8. Zusammensetzung gemäß Anspruch 1, wobei die Pheromone besagter Zusammensetzung wenigstens eine Fettsäure oder ein Derivat, vorzugsweise ein Ester-Derivat, insbesondere Methylester davon umfassen.

9. Zusammensetzung gemäß Anspruch 8, wobei die Fettsäure ausgewählt ist aus: Oleinsäure, Palmitinsäure, n-Buttersäure, Linolsäure, Palmitoleinsäure, Caprinsäure, Laurinsäure, Myristinsäure, Pentadecansäure, Stearinsäure, Azelainsäure, Pimelinsäure, Linolensäure, Pentansäure, Arachidonsäure, Isobuttersäure, Pivalinsäure, Eicosapentonsäure, Tridecansäure, Docosahexaensäure und einem Gemisch davon.

10. Zusammensetzung gemäß einem der Ansprüche 8 und 9, wobei besagte Zusammensetzung 0,01 Gew.-% bis ungefähr 5 Gew.-% Fettsäure oder Derivat davon bezogen auf das Gesamtgewicht der Zusammensetzung umfasst.

11. Zusammensetzung gemäß einem der Ansprüche 1 bis 10 für eine Verwendung in der Prävention und/oder Behandlung wenigstens eines stressbedingten Verhaltensproblems oder medizinischen Problems eines nicht-humanen Säugetiers.

12. Zusammensetzung für eine Verwendung gemäß Anspruch 11, wobei das stressbedingte Verhaltensproblem aus Urinmarkieren, Aggressivität, Kratzen, übermäßigem Putzen, Territorialkonflikten, Verringerung von Spieltrieb und sozialen Kontakten, Hyperagitation, Prostration und Destruktionen ausgewählt ist.

13. Zusammensetzung für eine Verwendung gemäß Anspruch 11, wobei das stressbedingte medizinische Problem aus Zystitis, Hautproblemen, Alopezia, Anorexie, Obesität, Diarrhö und Atemwegsinfektionen ausgewählt ist.

14. Kit umfassend eine Zusammensetzung gemäß einem der Ansprüche 1 bis 10 und/oder eine Vorrichtung, die für die Diffusion besagter Zusammensetzung an die Umgebungsluft geeignet ist, und gegebenenfalls eine Gebrauchsanleitung, zur Prävention und/oder Behandlung wenigstens eines stressbedingten Verhaltensproblems oder medizinischen Problems bei einem nicht-humanen Säugetier.

## Claims

1. A pheromone-based composition comprising at least one carrier solvent **characterized by** the following properties:
- a vapor pressure (VP) of between 1 and 10 Pa (measured at 20 or 25°C), and
- a flash point of greater than or equal to 95°C, wherein the carrier solvent comprises at least one dibasic ester of formula (I) 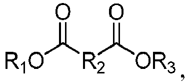 wherein R₁, R₂ and R₃ are independently linear or branched alkyl groups comprising from 1 to 10 carbon atoms.

2. The composition as claimed in claim 1, wherein R₁ and R₃ are identical.

3. The composition as claimed in claim 2, wherein R₁ and R₃ are isobutyl groups.

4. The composition as claimed in any one of claims 1-3, wherein the dibasic ester is chosen from dimethyl 2-methylpentanedioate, diisobutyl hexanedioate, diisobutyl glutarate, diisobutyl succinate and at least one mixture thereof.

5. The composition as claimed in any one of claims 1 to 4, wherein the composition comprises between 50 and 99.9% of carrier solvent, in particular dibasic ester, by weight relative to the total weight of the composition.

6. The composition as claimed in any one of claims 1 to 4, wherein the composition comprises between 95 and 97%, in particular dibasic ester, by weight relative to the total weight of the composition.

7. The composition as claimed in any one of claims 1 to 4, wherein the composition comprises 96.5%, in particular dibasic ester, by weight relative to the total weight of the composition.

8. The composition as claimed in claim 1, wherein the pheromones of said composition comprise at least one fatty acid or a derivative, preferably an ester derivative, in particular methyl ester, thereof.

9. The composition as claimed in claim 8, wherein the fatty acid is chosen from: oleic acid, palmitic acid, n-butyric acid, linoleic acid, palmitoleic acid, capric acid, lauric acid, myristic acid, pentadecanoic acid, stearic acid, azelaic acid, pimelic acid, linolenic acid, pentanoic acid, arachidonic acid, isobutyric acid, pivalic acid, eicosapentaenoic acid, tridecanoic acid, docosahexaenoic acid and a mixture thereof.

10. The composition as claimed in either one of claims 8 and 9, said composition comprising 0.01% to about 5% by weight of fatty acid or of derivative thereof, relative to the total weight of the composition.

11. The composition as claimed in any one of claims 1 to 10, for a use in the prevention and/or treatment of at least one behavioural or medical problem linked to stress in a non-human mammal.

12. The composition for a use as claimed in claim 11, wherein the behavioural problem linked to stress is chosen from urine marking, aggressiveness, scratching, excessive grooming, territorial conflicts, reduced play and social contact, hyperagitation, prostration and destruction.

13. The composition for a use as claimed in claim 11, wherein the medical problem linked to stress is chosen from cystitis, dermatological problems, alopecia, anorexia, obesity, diarrhoea and respiratory infections.

14. A kit comprising a composition as claimed in any one of claims 1 to 10 and/or a device suitable for diffusion of said composition into the ambient air, and optionally instructions for use, intended to prevent and/or treat at least one behavioural or medical problem linked to stress in a non-human mammal.
